(19) **Europäisches Patentamt**
**European Patent Office**
**Office européen des brevets**

(11) **EP 0 621 772 B1**

(12) **EUROPÄISCHE PATENTSCHRIFT**

(45) Veröffentlichungstag und Bekanntmachung des Hinweises auf die Patenterteilung:
**11.06.1997 Patentblatt 1997/24**

(21) Anmeldenummer: 93920839.3

(22) Anmeldetag: **30.09.1993**

(51) Int. Cl.$^6$: **A61K 7/06**, A61K 7/11

(86) Internationale Anmeldenummer:
**PCT/EP93/02661**

(87) Internationale Veröffentlichungsnummer:
**WO 94/12147 (09.06.1994 Gazette 1994/13)**

(54) **MITTEL ZUR FESTIGUNG DER HAARE**

HAIR FIXER

AGENT DE FIXATION CAPILLAIRE

(84) Benannte Vertragsstaaten:
**DE ES FR GB IT**

(30) Priorität: **25.11.1992 DE 4239499**

(43) Veröffentlichungstag der Anmeldung:
**02.11.1994 Patentblatt 1994/44**

(73) Patentinhaber: **Wella Aktiengesellschaft**
**64274 Darmstadt (DE)**

(72) Erfinder:
• **STARKE, Thomas**
**D-64367 Mühltal-Trautheim (DE)**
• **RAMGE, Christoph**
**D-65428 Rüsselsheim (DE)**
• **WOLFRAMM, Claus-Peter**
**D-64293 Darmstadt (DE)**
• **SCHAAFF, Günter**
**D-64295 Darmstadt (DE)**

(56) Entgegenhaltungen:
EP-A- 0 116 207      WO-A-91/15187
WO-A-93/09757      DE-A- 2 263 495
DE-A- 3 518 847

**Beschreibung**

Die Erfindung betrifft ein Mittel zur Festigung der Haare, welches eine Kombination aus mindestens einem Vinylpyrrolidon-Vinylacetat-Copolymeren und mindestens einem Vinylacetat-Vinylpropionat-Crotonsäure-Terpolymeren enthält.

Mittel zur Festigung der Haare bestehen üblicherweise aus Lösungen von filmbildenden natürlichen oder synthetischen Polymeren.

Aus ökologischen Gründen wurden die bis etwa 1986 für Mittel zur Festigung der Haare üblichen Treibmittel Fluorkohlenwasserstoffe und Methylenchlorid durch die umweltverträglicheren halogenfreien Kohlenwasserstoffe ersetzt.

Durch die Einführung der halogenfreien Kohlenwasserstoffe als Treibmittel ergab sich die Problematik, daß mit Fluorkohlenwasserstoffen oder Methylenchlorid als Treibmittel in Mitteln zur Festigung der Haare erprobte Polymere und Polymerkombinationen in den halogenfreie Kohlenwasserstoffe als Treibmittel enthaltenden Mitteln nicht anwendbar waren, da diese Polymere in den halogenfreie Kohlenwasserstoff-Treibmittel enthaltenden Mitteln nicht löslich waren und als Niederschlag ausfielen.

Auch mit der Einführung von Gemischen aus Dimethylether und halogenfreien Kohlenwasserstoffen als Treibmittel für Mittel zur Festigung der Haare ist es bisher nicht gelungen, ein Mittel zur Verfügung zu stellen, das in allen Eigenschaften -insbesondere aber bezüglich der Homogenität, der Auswaschbarkeit und der Festigungswirkung des Mittels - völlig zufriedenstellend ist.

Die Verwendung weniger polarer Polymere führte zwar zu einer Verbesserung der Verträglichkeit der Mittel zur Festigung der Haare mit halogenfreien Kohlenwasserstoff-Treibmitteln, insbesondere mit Propan/Butan-Gemischen. Jedoch konnten diese Mittel hinsichtlich der anwendungstechnischen Anforderungen, die an ein Mittel zur Festigung der Haare zu stellen sind, insbesondere bezüglich der Auswaschbarkeit, nicht befriedigen.

Es bestand daher die Aufgabe, ein Mittel zur Festigung der Haare zur Verfügung zu stellen, das die Nachteile der bisher bekannten, Fluorkohlenwasserstoff- und Methylenchlorid-freien Mittel zur Festigung der Haare nicht aufweist, das heißt mit halogenfreien Kohlenwasserstoffen als Treibmitteln verträglich ist, eine gute Festigung der Haare bewirkt ohne einen klebrigen Griff zu erzeugen, und insbesondere leicht aus dem Haar ausgewaschen werden kann.

Überraschenderweise wurde nunmehr gefunden, daß durch die Kombination von mindestens einem Vinylpyrrolidon-Vinylacetat-Copolymer mit mindestens einem Vinylacetat-Vinylpropionat-Crotonsäure-Terpolymer, ein Mittel zur Festigung der Haare zur Verfügung gestellt wird, das alle an ein derartiges Mittel gestellten Anforderungen in hervorragender Weise erfüllt.

Gegenstand der vorliegenden Erfindung ist daher ein Mittel zur Festigung der Haare auf der Basis einer 1 bis 12 Gewichtsprozent einer Polymerkombination enthaltenden alkoholischen oder wäßrig-alkoholischen Lösung, welches dadurch gekennzeichnet ist, daß die Polymerkombination aus a) mindestens einem Vinylpyrrolidon-Vinylacetat-Copolymer und b) mindestens einem zu 60 bis 100 Prozent neutralisierten Vinylacetat-Vinylpropionat-Crotonsäure-Terpolymer besteht, wobei das Copolymer a) und das Terpolymer b) in einem Gewichtsverhältnis von 1 : 9 bis 9 : 1 enthalten sind.

Vorzugsweise sind das Copolymer a) und das Terpolymer b) in dem erfindungsgemäßen Mittel in einem Gewichtsverhältnis von 2 : 8 bis 8 : 2 enthalten.

Von den Vinylpyrrolidon-Vinylacetat-Copolymeren a), die in dem erfindungsgemäßen Mittel enthalten sein können, sind jene bevorzugt, die ein Gewichtsverhältnis der Vinylpyrrolidon- zu den Vinylacetat-Monomeren von 20 : 80 bis 50 : 50 aufweisen und beispielsweise von der Firma BASF, Ludwigshafen/BRD unter den Handelsbezeichnungen Luviskol® VA 28, Luviskol® VA 37 und Luviskol® VA 55 vertrieben werden.

Von den Vinylpyrrolidon-Vinylacetat-Copolymeren a), die bevorzugt in dem erfindungsgemäßen Mittel enthalten sein können, ist das Polymer mit einem Gewichtsverhältnis der Vinylpyrrolidon- zu den Vinylacetat-Monomeren von 30 : 70, das beispielsweise von der Firma BASF, Ludwigshafen/BRD, unter der Handelsbezeichnung Luviskol® VA 37 und in Form einer 50%igen Lösung in Isopropanol unter der Handelsbezeichnung Luviskol® VA 37 I vertrieben wird, besonders bevorzugt.

Von den Vinylacetat-Vinylpropionat-Crotonsäure-Terpolymeren b), die in dem erfindungsgemäßen Mittel enthalten sein können, ist jenes bevorzugt, das ein Gewichtsverhältnis der Vinylacetat-, Vinylpropionat- und Crotonsäure-Monomeren von 50 : 40 : 10 aufweist und beispielsweise von der Firma BASF, Ludwigshafen/BRD, unter der Handelsbezeichnung Luviset® CAP vertrieben wird.

Das Terpolymer b) wird in einer zu 60 bis 100 Prozent durch organische Amine oder Alkanolamine neutralisierten Form eingesetzt werden.

Zur Neutralisierung des Terpolymers b) geeignete Amine oder Alkanolamine sind zum Beispiel

2-Amino-2-methyl-1,3-propandiol,
2-Amino-2-ethyl-1,3-propandiol,
2-Amino-2-methyl-1-propanol,
2-Amino-2-methyl-2-pentanol,

1-Amino-2-methyl-2-propanol,

2-Amino-1-butanol,

3-Amino-2-pentanol,

2-Amino-1-phenyl-1-butanol,

2-Dimethylamino-2-methyl-1-propanol,

2-Dimethylamino-2-methyl-1,3-propandiol,

tris-(Hydroxymethyl)-aminomethan,

tris-(Hydroxymethyl)-dimethylaminomethan,

und

$N^1$-(2-Hydroxyethyl)-2-methyl-1,2-propandiamin,

wobei eine Neutralisierung mit 2-Amino-2-methyl-1-propanol, 2-Amino-1-butanol und 2-Amino-2-methyl-1,3-propandiol besonders bevorzugt ist.

Das Copolymer a) und das Terpolymer b) sind in dem erfindungsgemäßen Mittel jeweils in einer Menge von 0,5 bis 10 Gewichtsprozent, vorzugsweise von 0,5 bis 6 Gewichtsprozent, enthalten, wobei die Gesamtmenge der Polymerkombination aus Vinylpyrrolidon-Vinylacetat-Copolymer a) und Vinylacetat-Vinylpropionat-Crotonsäure-Terpolymer b) in dem erfindungsgemäßen Mittel bevorzugt 2 bis 10 Gewichtsprozent beträgt.

Das erfindungsgemäße Mittel zur Festigung der Haare weist vorzugsweise einen pH-Wert von 5 bis 9,5 auf.

Als Alkohol enthält das erfindungsgemäße Mittel insbesondere die für kosmetische Zwecke üblicherweise verwendeten niederen Alkohole mit 1 bis 4 Kohlenstoffatomen, wie zum Beispiel Ethanol und Isopropanol. Diese Alkohole können in dem erfindungsgemäßen Mittel in einer Menge von 15 bis 85 Gewichtsprozent, vorzugsweise in einer Menge von 25 bis 75 Gewichtsprozent, enthalten sein.

Das erfindungsgemäße Mittel kann einen Wassergehalt von bis zu 10 Gewichtsprozent aufweisen, wobei ein Wassergehalt von 2 bis 8 Gewichtsprozent bevorzugt ist.

Selbstverständlich können in dem erfindungsgemäßen Mittel auch weitere haarkosmetische Zusätze, wie beispielsweise Parfümöle, Kräuterextrakte, bakterizide oder fungizide Stoffe, Lösungsvermittler für Parfümöle, plastifizierende Zusätze wie Phthalsäureester oder Alkylzitrate, oder hydrophobierende Substanzen wie Siliconöle, in einer Gesamtmenge von bis zu 1 Gewichtsprozent enthalten sein, soweit derartige Zusätze zweckmäßig erscheinen.

Das erfindungsgemäße Mittel zur Festigung der Haare wird vorzugsweise unter Zusatz eines Treibmittels in einem Druckbehälter abgefüllt und liegt als Aerosol-Haarspray oder Aerosol-Haarlack vor.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einen Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$, sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Das Mittel zur Festigung der Haare gemäß der vorliegenden Erfindung ist mit halogenfreien Kohlenwasserstoffen als Treibmittel hervorragend verträglich, bewirkt eine ausgezeichnete Festigung ohne einen klebrigen Griff des Haares zu erzeugen, ist gut aus dem Haar ausbürstbar und leicht aus dem Haar auswaschbar.

Die nachfolgenden Beispiele sollen den Gegenstand der Erfindung näher erläutern:

## Beispiele

## Beispiel 1: Aerosol-Haarspray

6,00 g      Vinylpyrrolidon-Vinylacetat-Copolymer, 50%ige Lösung in Isopropanol, (Luviskol® VA 37I der Firma BASF/BRD)

2,20 g      Vinylacetat-Vinylpropionat-Crotonsäure-Terpolymer (Luviset® CAP der Firma BASF/BRD, zu 95 % mit 2-Amino-2-methyl-1-propanol neutralisiert)

0,20 g      Parfümöl

| | |
|---|---|
| 0,10 g | Phenylmethylsiloxan |
| 0,40 g | Diethylphthalat |
| 35,10 g | Ethanol |
| 22,00 g | Propan/Butan |
| 34,00 g | Dimethylether |
| 100,00 g | |

**Beispiel 2: Aerosol-Haarspray**

| | |
|---|---|
| 6,00 g | Vinylpyrrolidon Vinylacetat-Copolymer, 50%ige Lösung in Isopropanol, (Luviskol$^®$ VA 37I der Firma BASF/BRD) |
| 2,20 g | Vinylacetat-Vinylpropionat-Crotonsäure-Terpolymer (Luviset$^®$ CAP der Firma BASF/BRD, zu 95 % mit 2-Amino-2-methyl-1-propanol neutralisiert |
| 0,20 g | Parfümöl |
| 0,30 g | Diethylphthalat |
| 28,30 g | Ethanol |
| 7,00 g | Wasser |
| 22,00 g | Propan/Butan |
| 34,00 g | Dimethylether |
| 100,00 g | |

**Beispiel 3: Aerosol-Haarspray**

| | |
|---|---|
| 3,60 g | Vinylpyrrolidon-Vinylacetat-Copolymer, 50%ige Lösung in Isopropanol, (Luviskol$^®$ VA 37I der Firma BASF/BRD) |
| 3,10 g | Vinylacetat-Vinylpropionat-Crotonsäure-Terpolymer (Luviset$^®$ CAP der Firma BASF/BRD, zu 100 % mit 2-Amino-2-methyl-1-propanol neutralisiert) |
| 0,20 g | Parfümöl |
| 0,40 g | Diethylphthalat |
| 36,70 g | Ethanol |
| 22,00 g | Propan/Butan |
| 34,00 g | Dimethylether |
| 100,00 g | |

**Beispiel 4: Aerosol-Haarspray**

| | |
|---|---|
| 5,00 g | Vinylpyrrolidon-Vinylacetat-Copolymer, 50%ige Lösung in Isopropanol, (Luviskol$^®$ VA 37I der Firma BASF/BRD) |
| 2,80 g | Vinylacetat-Vinylpropionat-Crotonsäure-Terpolymer (Luviset$^®$ CAP der Firma BASF/BRD, zu 100 % mit 2-Amino-2-methyl-1-propanol neutralisiert) |
| 0,20 g | Parfümöl |
| 0,60 g | Diethylphthalat |
| 59,40 g | Ethanol |
| 4,00 g | Wasser |
| 28,00 g | Propan/Butan |
| 100,00 g | |

**Beispiel 5: Non-Aerosol-Haarspray**

| | |
|---|---|
| 6,20 g | Vinylpyrrolidon-Vinylacetat-Copolymer, 50%ige Lösung in Isopropanol, (Luviskol$^®$ VA 37I der Firma BASF/BRD) |
| 3,40 g | Vinylacetat-Vinylpropionat-Crotonsäure-Terpolymer (Luviset$^®$ CAP der Firma BASF/BRD, zu 95 % mit 2-Amino-2-methyl-1-propanol neutralisiert) |
| 0,30 g | Parfümöl |
| 1,20 g | Diethylphthalat |
| 81,90 g | Ethanol |
| 7,00 g | Wasser |
| 100,00 g | |

**Vergleichsversuche**

Das erfindungsgemäße Aerosolhaarspray gemäß Beispiel 1 wurde bezüglich seiner Festigungseigenschaften, seiner Klebrigkeit, seiner Ausbürstbarkeit und seiner Auswaschbarkeit mit drei nicht-erfindungsgemäßen Haarsprays A, B und C verglichen, die jeweils nur ein Vinylacetat-Vinylpropionat-Crotonsäure-Terpolymer enthalten.

Vergleichsversuche mit einem nicht-erfindungsgemäßen Mittel, das anstelle des im Haarspray A enthaltenen Vinylacetat-Vinylpropionat-Crotonsäure-Terpolymers die gleiche Menge an Vinylpyrolidon-Vinylacetat-Copolymer enthält, werden im folgenden nicht beschrieben, da Vorversuche ergaben, daß ein derartiges nicht-erfindungsgemäßes Mittel schon hinsichtlich der für Haarsprays bedeutenden Festigungsstärke ein völlig ungenügendes Ergebnis zeigt.

**Haarspray A**

| | |
|---|---|
| 4,20 g | Vinylacetat-Vinylpropionat-Crotonsäure-Terpolymer (Luviset[®] CAP der Firma BASF/BRD, zu 95 % mit 2-Amino-2-methyl-1-propanol neutralisiert) |
| 0,20 g | Parfümöl |
| 0,10 g | Phenylmethylsiloxan |
| 0,30 g | Diethylphthalat |
| 31,20 g | Ethanol |
| 24,00 g | Propan/Butan |
| 40,00 g | Dimethylether |
| 100,00 g | |

**Haarspray B**

| | |
|---|---|
| 4,20 g | Vinylacetat-Vinylpropionat-Crotonsäure-Terpolymer (Luviset[®] CAP der Firma BASF/BRD, zu 95 % mit 2-Amino-2-methyl-1-propanol neutralisiert) |
| 0,20 g | Parfümöl |
| 0,10 g | Phenylmethylsiloxan |
| 0,40 g | Diethylphthalat |
| 39,10 g | Ethanol |
| 22,00 g | Propan/Butan |
| 34,00 g | Dimethylether |
| 100,00 g | |

**Haarspray C**

| | |
|---|---|
| 5,20 g | Vinylacetat-Vinylpropionat-Crotonsäure-Terpolymer (Luviset[®] CAP der Firma BASF/BRD, zu 95 % mit 2-Amino-2-methyl-1-propanol neutralisiert) |
| 0,20 g | Parfümöl |
| 0,10 g | Phenylmethylsiloxan |
| 0,40 g | Diethylphthalat |
| 38,10 g | Ethanol |
| 22,00 g | Propan/Butan |
| 34,00 g | Dimethylether |
| 100,00 g | |

In einem Anwendungstest auf frisiertem Haar wurden die 3 nicht-erfindungsgemäßen Haarspray-Präparate und das erfindungsgemäße Haarspray gemäß Beispiel 1 zunächst hinsichtlich ihrer Festigungsstärke und der Klebrigkeit des Griffs der mit diesen Haarsprays besprühten Haare und sodann hinsichtlich ihrer Ausbürstbarkeit und ihrer Auswaschbarkeit miteinander verglichen.

Hierzu wurden die Haare von 100 Versuchspersonen aus einer Entfernung von 30 cm mit dem Haarspray unter Verwendung eines üblichen Aerosolventil-Sprühkopf-Systems (mittlere Ausbringungsrate: 0,8 g/s) 10 Sekunden lang besprüht und zunächst hinsichtlich der Festigungsstärke, der Haltbarkeit der Frisur und der Klebrigkeit des Griffs der Haare beurteilt.

Die in den nachfolgenden Tabellen 1 bis 4 angegebenen Bewertungen stellen den aus den Einzelbewertungen erhaltenen Mittelwert dar.

Tabelle 1

| Festigung | | |
|---|---|---|
| | Festigungsstärke | Haltbarkeit der Frisur |
| Haarspray gemäß Beispiel 1 | sehr gut bis gut | sehr gut bis gut |
| Haarspray A | gut | gut |
| Haarspray B | gut | gut |
| Haarspray C | sehr gut | sehr gut |

Tabelle 2

| Klebrigkeit | |
|---|---|
| | Klebrigkeit |
| Haarspray gemäß Beispiel 1 | nicht klebrig |
| Haarspray A | klebrig |
| Haarspray B | leicht klebrig |
| Haarspray C | klebrig |

**Ausbürstbarkeit**

Die Ausbürstbarkeit der 4 Haarsprays wurde getestet, in dem das frisierte, zuvor mit jeweils einem der 4 Haarsprays aus einer Entfernung von 30 cm unter Verwendung eines üblichen Aerosolventil-Sprühkopf-Systems (Ausbringungsrate: 0,8 g/s) 10 Sekunden lang eingesprühte Haar der Versuchspersonen mit einer üblichen Haarbürste 5 Minuten lang durchgebürstet und anschließend anhand von Griff und Weichheit der Haare sowie auf das Vorhandenseins von sichtbaren Rückständen beurteilt wurde.

Tabelle 3

| Ausbürstbarkeit | |
|---|---|
| | Ausbürstbarkeit |
| Haarspray gemäß Beispiel 1 | gut |
| Haarspray A | befriedigend |
| Haarspray B | befriedigend |
| Haarspray C | ausreichend |

**Auswaschbarkeit**

Die Auswaschbarkeit der 3 nicht-erfindungsgemäßen Haarsprays und des erfindungsgemäßen Haarsprays gemäß Beispiel 1 wurde getestet, in dem das frisierte Haar der Versuchspersonen mehrmals mit einem der 4 Haarsprays aus einer Entfernung von 30 cm unter Verwendung eines üblichen Aerosolventil-Sprühkopf-Systems (Ausbringungsrate: 0,8 g/s) eingesprüht und zwischendurch getrocknet wurde.

Das Haar der Versuchspersonen wurde sodann in üblicher Weise mit 35 °Celsius warmem Wasser jeweils unter Verwendung von 6 g eines üblichen Haarshampoos gewaschen.

Im Anschluß an diese Haarwäsche wurden die Haare der Versuchspersonen getrocknet und die Auswaschbarkeit der 3 nicht-erfindungsgemäßen Haarsprays A, B und C und des erfindungsgemäßen Haarsprays gemäß Beispiel 1 anhand von Griff und Weichheit der Haare sowie des Vorhandenseins von sichtbaren Rückständen beurteilt.

Die in der nachfolgenden Tabelle 4 angegebenen Bewertungen stellen den aus Einzelbewertungen erhaltenen Mittelwert dar.

Tabelle 4

| Auswaschbarkeit | |
|---|---|
| | Auswaschbarkeit |
| Haarspray gemäß Beispiel 1 | sehr gut |
| Haarspray A | befriedigend bis gut |
| Haarspray B | gut |
| Haarspray C | ausreichend |

Durch die vorstehenden Vergleichsversuche wird eindeutig belegt, daß das erfindungsgemäße Haarspray gegenüber den nicht-erfindungsgemäßen Haarsprays A, B und C bezüglich der Klebrigkeit des Griffs und der Ausbürstbarkeit, insbesondere jedoch bezüglich der Auswaschbarkeit, bei vergleichbaren Festigungseigenschaften, deutliche Vorteile aufweist.

Die hervorragende Auswaschbarkeit der in dem erfindungsgemäßen Mittel enthaltenen Polymerkombination zeigt sich insbesondere durch den Vergleich des erfindungsgemäßen Haarsprays 1 mit einem Gehalt an der Polymerkombination aus den Polymeren a) und b) und dem nicht-erfindungsgemäßen Beispiel C, das die der Menge der Polymerkombination aus a) und b) entsprechende Menge an Terpolymer b) (bezogen auf die Festsubstanz) enthält.

Alle Prozentangaben in der vorliegenden Anmeldung stellen Gewichtsprozente dar.

## Patentansprüche

1. Mittel zur Festigung der Haare auf der Basis einer 1 bis 12 Gewichtsprozent einer Polymerkombination enthaltenden alkoholischen oder wäßrig-alkoholischen Lösung, dadurch gekennzeichnet, daß die Polymerkombination aus a) mindestens einem Vinylpyrrolidon-Vinylacetat-Copolymer und b) mindestens einem zu 60 bis 100 Prozent neutralisierten Vinylacetat-Vinylpropionat-Crotonsäure-Terpolymer besteht, wobei das Copolymer a) und das Terpolymer b) in einem Gewichtsverhältnis von 1 : 9 bis 9 : 1 enthalten sind.

2. Mittel nach Anspruch 1, dadurch gekennzeichnet, daß es das Copolymer a) in einer Menge von 0,5 bis 10 Gewichtsprozent enthält.

3. Mittel nach einem der Ansprüche 1 oder 2, dadurch gekennzeichnet, daß es das Terpolymer b) in einer Menge von 0,5 bis 10 Gewichtsprozent enthält.

4. Mittel nach einem der Ansprüche 1 bis 3, dadurch gekennzeichnet, daß das Gewichtsverhältnis von Copolymer a) zu Terpolymer b) 2 : 8 bis 8 : 2 beträgt.

5. Mittel nach einem der Ansprüche 1 bis 4, dadurch gekennzeichnet, daß das Terpolymer b) mit 2-Amino-2-methyl-1,3-propandiol, 2-Amino-2-ethyl-1,3-propandiol, 2-Amino-2-methyl-1-propanol, 2-Amino-2-methyl-2-pentanol, 1-Amino-2-methyl-2-propanol, 2-Amino-1-butanol, 3-Amino-2-pentanol, 2-Amino-1-phenyl-1-butanol, 2-Dimethylamino-2-methyl-1-propanol, 2-Dimethylamino-2-methyl-1,3-propandiol, tris-(Hydroxymethyl)-dimethylaminomethan, tris-(Hydroxymethyl)-aminomethan oder N[1]-(2-Hydroxyethyl)-2-methyl-1,2-propandiamin neutralisiert ist.

6. Mittel nach einem der Ansprüche 1 bis 5, dadurch gekennzeichnet, daß es 15 bis 85 Gewichtsprozent eines Treibmittels enthält und in Form eines Aerosol-Haarsprays oder Aerosol-Haarlackes vorliegt.

7. Mittel nach einem der Ansprüche 1 bis 6, dadurch gekennzeichnet, daß es in Form eines Non-Aerosol-Haarsprays oder Non-Aerosol-Haarlackes vorliegt.

**Claims**

1. Hair-setting agent based on an alcoholic or aqueous-alcoholic solution containing from 1 to 12 weight % of a polymer combination, characterised in that the polymer combination consists of a) at least one vinylpyrrolidone-vinyl acetate copolymer and b) at least one vinyl acetate-vinylproprionate-crotonic acid terpolymer neutralized to 60 to 100%, the copolymer a) and the terpolymer b) being present in a weight ratio of from 1:9 to 9:1.

2. Agent according to Claim 1, characterised in that it contains the copolymer a) in an amount of from 0.5 to 10 weight %.

3. Agent according to either of Claims 1 and 2, characterised in that it contains the terpolymer b) in an amount of from 0.5 to 10 weight %.

4. Agent according to any one of Claims 1 to 3, characterised in that the weight ratio of copolymer a) to terpolymer b) is from 2:8 to 8:2.

5. Agent according to any one of Claims 1 to 4, characterised in that the terpolymer b) is neutralised with 2-amino-2-methyl-1,3-propane diol, 2-amino-2-ethyl-1,3-propane diol, 2-amino-2-methyl-1-propanol, 2-amino-2-methyl-2-pentanol, 1-amino-2-methyl-2-propanol, 2-amino-1-butanol, 3-amino-2-pentanol, 2-amino-1-phenyl-1-butanol, 2-dimethylamino-2-methyl-1-propanol, 2-dimethylamino-2-methyl-1,3-propane diol, tris-(hydroxymethyl)-dimethylaminomethane, tris-(hydroxymethyl)-aminomethane or $N^1$-(2-hydroxyethyl)-2-methyl-1,2-propane diamine.

6. Agent according to any one of Claims 1 to 5, characterised in that it contains from 15 to 85 weight % of a propellant and is in the form of an aerosol hair spray or aerosol hair lacquer.

7. Agent according to any one of Claims 1 to 6, characterised in that it is in the form of a non-aerosol hair spray or non-aerosol hair lacquer.

**Revendications**

1. Produit de fixation des cheveux, à base d'une solution alcoolique ou aqueuse-alcoolique contenant de 1 à 12 % en poids d'une combinaison polymère, caractérisé en ce que la combinaison polymère est composée de a) au moins un copolymère vinylpyrrolidoneacétate de vinyle et b) au moins un terpolymère acétate de vinyle-propionate de vinyle-acide crotonique neutralisé à concurrence de 60 à 100 %, le copolymère a) et le terpolymère b) étant contenus en un rapport de poids de 1 : 9 à 9 : 1.

2. Produit selon la revendication 1, caractérisé en ce qu'il contient le copolymère a) en une quantité de 0,5 à 10 % en poids.

3. Produit sel on l'une des revendications 1 ou 2, caractérisé en ce qu'il contient le terpolymère b) en une quantité de 0,5 à 10 % en poids.

4. Produit sel on l'une des revendications 1 à 3, caractérisé en ce que le rapport de poids entre le copolymère a) et le terpolymère b) est de 2 : 8 à 8 : 2.

5. Produit sel on l'une des revendications 1 à 4, caractérisé en ce que le terpolymère b) est neutralisé au 2-amino-2-méthyl-1,3-propanediol, 2-amino-2-éthyl-1,3-propanediol, 2-amino-2-méthyl-1-propanol, 2-amino-2-méthyl-2-pentanol, 1-amino-2-méthyl-2-propanol, 2-amino-1-butanol, 3-amino-2-pentanol, 2-amino-1-phényl-1-bunatol, 2-diméthylamino-2-méthyl-1-propanol, 2-diméthylamino-2-méthyl-1,3-propanediol, tris-(hydroxyméthyl)-diméthylaminométhane, tris-(hydroxyméthyl)-aminométhane ou $N^1$-(2-hydroxyéthyl)-2-méthyl-1,2-propanediamine.

6. Produit selon l'une des revendications 1 à 5, caractérisé en ce qu'il contient de 15 à 85 % en poids d'un agent soufflant et se présente sous la forme d'un spray pour cheveux aérosol ou d'une laque pour cheveux en aérosol.

7. Produit selon l'une des revendications 1 à 6, caractérisé en ce qu'il se présente sous la forme d'un spray pour cheveux non aérosol ou d'une laque pour cheveux non aérosol.